# EUROPEAN PATENT APPLICATION

(11) **EP 3 689 333 A1**
(43) Date of publication of application: **05.08.2020**
(21) Application number: 18858885.9
(22) Date of filing: 19.09.2018
(51) Int. Cl.: A61K 9/06, A61K 47/36, A61K 47/42, G01N 33/15, G01N 33/50

(54) **TWO-DRUG TYPE LOCAL INJECTION SOLUTION FOR SUBMUCOSAL INJECTION**

(30) Priority: 25.09.2017 JP 2017183841
(71) Applicant: Kyoto Prefectural Public University Corporation, Kyoto-shi, Kyoto 602-8566 (JP)
(72) Inventor: HIROSE, Ryohei, Kyoto-shi Kyoto 602-8566 (JP); NAKAYA, Takaaki, Kyoto-shi Kyoto 602-8566 (JP); ITOH, Yoshito, Kyoto-shi Kyoto 602-8566 (JP)
(74) Representative: Winter, Brandl, Fürniss, Hübner, Röss, Kaiser, Polte - Partnerschaft mbB
(86) International application number: PCT/JP2018/034672
(87) International publication number: WO 2019/059237

(57) **Abstract**

This invention provides a two-component local injection solution for submucosal injection, comprising the following (i) and (ii):
(i) a polysaccharide aqueous solution containing at least one member selected from the group consisting of sodium alginate, carrageenan, and gellan gum; and
(ii) an aqueous salt solution containing at least one cation selected from the group consisting of sodium ions and calcium ions,
wherein when the polysaccharide is sodium alginate, the cation comprises calcium ions.

## Description

### Technical Field

The present invention relates to a two-component local injection solution for submucosal injection, and use in the manufacture of the local injection solution.

In this specification, the following abbreviations may be used:
HA: sodium hyaluronate
G-LA: gellan gum
SA: sodium alginate
Agar: carrageenan
SEH: submucosal elevation height
NS: saline
The expression "%" represents "percentage by mass."

### Background Art

In recent years, the number of endoscopic treatments for gastrointestinal cancers (stomach cancer, esophagus cancer, and colon cancer) has been increasing year by year. At the same time, difficult endoscopic treatment cases (a large tumor size, a submucosal layer with fibrosis, a tumor at a difficult-to-treat site) requiring advanced treatment techniques have also been increasing.

The endoscopic treatment currently performed in Japan for gastrointestinal cancers is based on two techniques, i.e., endoscopic mucosal resection (EMR) and endoscopic submucosal dissection (ESD). In both techniques, a very important step is "local injection" in which a local injection solution, such as saline, is injected into a submucosal layer to raise the mucous membrane.

Obtaining a sufficiently high mucosal elevation with local injection, and maintaining the mucosal elevation for a long period of time, are very important factors in practicing safe and reliable techniques (tumor resection). In particular, for difficult endoscopic treatment cases, the success or failure of the treatment itself depends on these factors. More specifically, in order to prevent gastrointestinal perforation, which is a serious complication of endoscopic treatment (caused by resection of/damage to not only the mucosal layer where the tumor is located, but also the muscular layer), if the mucosal layer can be lifted high with local injection, the distance between the muscular layer and the mucosal layer increases, which makes it possible to avoid damage to the muscular layer. Furthermore, if the mucosal elevation is high, snaring (strangulation) during EMR, or mucosal incision/dissection during ESD is easily performed; this improves accuracy and makes appropriate tumor resection possible without any residual tumor.

At present, 0.4% sodium hyaluronate (HA, product name: MucoUp), which is a local injection solution with a high viscosity, has been developed and is used clinically.

Further, as a replacement for MucoUp, local injection solutions have been proposed, such as a polysaccharide having a pseudoplastic viscosity (Patent Literature (PTL) 1), highly viscous substances or hydrophilic polymers, such as polyethylene glycol and sodium alginate (PTL 2), a biodegradable hydrogel (Non-patent Literature (NPL) 1), a polymer that changes from a liquid to a gel in a temperature-dependent manner (NPL 2), and photocrosslinkable chitosan (NPL 3). At the present stage, however, no excellent local injection solutions have been developed that can replace HA.

### Citation List

### Patent Literature

PTL 1: WO2013/077357
PTL 2: JP2007-75569A

### Non-patent Literature

NPL 1: Tran, R.T. et al., Gastrointest. Endosc. 75(5): 1092-1097, 2012.
NPL 2: Fernandez-Esparrach, G. et al., Gastrointest. Endosc. 69 (6): 1135-1139, 2009.
NPL 3: Hayashi, T. et al., J. Biomed. Mater. Res. B. Appl. Biomater. 71(2): 367-372, 2004.

### Summary of Invention

### Technical Problem

An object of the present invention is to provide a low-cost local injection solution by which high mucosal elevation is obtained, the elevation is maintained for a long period of time, and tissue is less likely to be damaged.

Another object of the present invention is to provide a local injection in which adjustments can be made, even when local injection fails.

### Solution to Problem

The present invention provides the following local injection.
Item 1. A two-component local injection solution for submucosal injection, comprising the following (i) and (ii):
   (i) a polysaccharide aqueous solution containing at least one member selected from the group consisting of sodium alginate, carrageenan, and gellan gum; and
   (ii) an aqueous salt solution containing at least one cation selected from the group consisting of sodium ions and calcium ions,
   wherein when the polysaccharide is sodium alginate, the cation comprises calcium ions.
Item 2. The two-component local injection solution for submucosal injection according to Item 1, wherein the polysaccharide concentration is 0.1 to 2% by mass.
Item 3. The two-component local injection solution for submucosal injection according to Item 1 or 2, wherein the salt concentration is 0.1 to 4 w/v%.
Item 4. The two-component local injection solution for submucosal injection according to any one of Items 1 to 3, wherein the polysaccharide is sodium alginate, and the aqueous salt solution contains calcium ions.
Item 5. The two-component local injection solution for submucosal injection according to any one of Items 1 to 3, wherein the polysaccharide is carrageenan or gellan gum, and the aqueous salt solution contains sodium ions or calcium ions.
Item 6. The two-component local injection solution for submucosal injection according to any one of Items 1 to 3 and 5, wherein the carrageenan comprises κ-carrageenan and/or ι-carrageenan.
Item 7. The two-component local injection solution for submucosal injection according to any one of items 1 to 6, which is for use in endoscopic mucosal resection (EMR) or endoscopic submucosal dissection (ESD).
Item 8. Use of a polysaccharide aqueous solution containing at least one member selected from the group consisting of sodium alginate, carrageenan, and gellan gum, in the manufacture of a two-component local injection solution for submucosal injection.
Item 9. Use of an aqueous salt solution containing at least one cation selected from the group consisting of sodium ions and calcium ions, in the manufacture of a two-component local injection solution for submucosal injection.

### Advantageous Effects of Invention

If high mucosal elevation is obtained with local injection, the mucosal layer can be lifted high, increasing the distance between the muscular layer and the mucosal layer; accordingly, damage to the muscular layer (gastrointestinal perforation) can be avoided. On the other hand, if the mucosal elevation is low, the distance between the muscular layer and the mucosal layer is short, and the risk of damage to the muscular layer (gastrointestinal perforation) or risk of tumors left behind in the mucosal layer increases. By developing and using a local injection solution with a higher viscosity, it is theoretically possible to increase the height of mucosal elevation with local injection. However, an increase in the viscosity of a local injection solution makes injection with a local injection needle in endoscopic treatment difficult in actuality. Taking these facts into account, currently used local injection solutions have a concentration and viscosity that are adjusted to achieve the highest performance (mucosal elevation) within the range in which injection in actual endoscopic treatment is possible without problems. (This is one of the reasons why the upper limit of HA concentration is currently 0.4%.)

The two-component local injection solution according to the present invention comprises a polysaccharide aqueous solution that gels with cations (Ca²⁺, Na⁺), and an aqueous salt solution that contains cations. When injected into the submucosal tissue, the two liquids are mixed and formed into a gel, greatly increasing the viscosity. In this manner, it is possible to obtain mucosal elevation with a local injection solution having a viscosity higher than the upper limit of injectable viscosity.

By the use of these two-component local injection solutions, high mucosal elevation can be obtained, the elevation can be maintained for a long period of time, and the damage to tissue can be reduced. Further, these two-component local injection solutions are inexpensive. By injecting the two components of the two-component local injection solution of the present invention, high mucosal elevation is obtained that cannot be obtained with a single-component injection. Furthermore, when elevation is not obtained at an expected mucosal site with the injection of the first injection solution (the polysaccharide aqueous solution or aqueous salt solution), the elevation will decrease after several minutes without the injection of the second injection solution (the aqueous salt solution or polysaccharide aqueous solution); therefore, the first injection solution can be re-injected, and the second injection solution can then be injected to obtain elevation at a target mucosal site. Accordingly, adjustments can be made when the local injection fails.

In the current use of 0.4% sodium hyaluronate (HA) as well, it is often the case that a saline is first locally injected, and after confirmation of the formation of mucosal elevation at an appropriate site, HA is locally injected. According to this method, the concentration and viscosity of HA may decrease due to the saline injected in the first local injection, which possibly deteriorates the performance. In the present invention, however, this is not a concern.

### Brief Description of Drawings

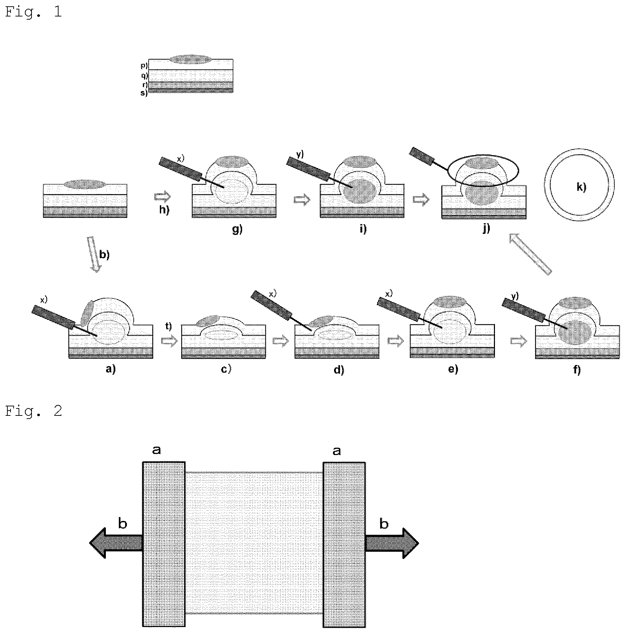

Fig. 1 is a schematic diagram of a two-step system using the local injection solution of the present invention. a) Local injection into an inappropriate site, b) failure of the local injection, c) spontaneous disappearance of elevation, d) local injection again, e) local injection into an appropriate site, f) reinforcing the elevation with additional local injection (increasing the height of elevation and increasing the time for maintaining the elevation by increasing the viscosity by mixing the two liquids), g) local injection into an appropriate site, h) success of local injection, i) reinforcing the elevation with additional local injection (increasing the elevation and increasing the time for maintaining the elevation by increasing the viscosity by mixing the two liquids), j) applying a snare, k) continuing treatment, x) first agent, y) second agent, p) mucosal layer, q) submucosal layer, r) muscular layer, s) serous membrane, t) after several minutes.
Fig. 2 shows an *ex vivo* model of the present invention, in which a digestive tract sample is fixed with clips. Uniform tension (b) is applied with two clips (a).
Fig. 3 is a graph showing the results of measurement of SEH in terms of sodium alginate over time.
Fig. 4 is a graph showing the results of measurement of SEH in terms of gellan gum over time.
Fig. 5 is a graph showing the results of measurement of SEH in terms of carrageenan over time.

### Description of Embodiments

The two-component local injection solution for submucosal injection of the present invention is used for injection into a submucosal layer to swell the mucosal layer and remove tumors etc. with an endoscopic knife, or by strangulation or application of a current with a snare. Examples of endoscopic treatment in which the local injection solution of the present invention is used include, but are not limited to, endoscopic mucosal resection (EMR) and endoscopic submucosal layer dissection (ESD).

The two-component local injection solution for submucosal injection according to the present invention comprises (i) a polysaccharide aqueous solution containing at least one member selected from the group consisting of sodium alginate, carrageenan, and gellan gum; and (ii) an aqueous salt solution containing at least one cation selected from the group consisting of sodium ions and calcium ions. These solutions are injected into a submucosal layer. The order of injection may be any order. The polysaccharide aqueous solution (i) may first be injected, and the aqueous salt solution (ii) may then be injected. It is also possible to first inject the aqueous salt solution (ii), and then inject the polysaccharide aqueous solution (i).

Below, the aqueous solution (i) or (ii) to be first injected into a submucosal layer may sometimes be referred to as "the first liquid," while the aqueous solution (ii) or (i) to be subsequently injected may sometimes be referred to as "the second liquid."

The local injection solution of the present invention can be injected, for example, into the submucosal layer of the digestive tract. As shown in Fig. 1, the digestive tract has four layers, i.e., p) mucosal layer, q) submucosal layer, r) muscular layer, and s) serous membrane. Both of the first liquid and second liquid of the local injection solution of the present invention are injected into the submucosal layer q).

In Fig. 1, a) when the first liquid x) is locally injected into an inappropriate site, and b) when the local injection fails, t) the site is allowed to stand for several minutes to c) allow spontaneous disappearance of the elevation. Thereafter, d) local injection is performed again to e) locally apply the first liquid x) to an appropriate site. Next, f) the second liquid y) is locally injected additionally to reinforce the elevation (increasing the height of the elevation, and increasing the time for maintaining the elevation). Thereafter, the tumor at the elevation site is excised with a snare j) or a knife, and k) the treatment is continued.

In one preferable embodiment, the polysaccharide aqueous solution, in particular, aqueous solutions of gellan gum and carrageenan, have a slightly higher viscosity; thus, if the injection of the first liquid containing polysaccharide into a submucosal layer fails, spontaneous disappearance of the elevation takes a slightly longer period of time. Therefore, it is advantageous to use the aqueous salt solution as the first liquid since spontaneous disappearance of the elevation occurs promptly, even when the local injection fails.

Carrageenan preferably comprises iota carrageenan. Iota carrageenan gels with calcium ions. Preferable carrageenan may be iota carrageenan alone; or a mixture of two or three carrageenan types, including kappa carrageenan and/or lambda carrageenan, together with iota carrageenan. Carrageenan is obtained from red algae, as is agar; thus, in the present specification and drawings, carrageenan may be referred to as "agar."

When sodium alginate, which gels with calcium ions, is used as polysaccharide, the aqueous salt solution contains calcium ions.

Gellan gum gels with both sodium ions and calcium ions. Therefore, when gellan gum is used as polysaccharide, the aqueous salt solution contains at least one cation selected from sodium ions and calcium ions.

Examples of salts for preparing the aqueous salt solution containing sodium ions or calcium ions include sodium chloride, calcium chloride, sodium lactate, calcium lactate, sodium citrate, calcium citrate, sodium malate, calcium malate, sodium gluconate, calcium gluconate, and the like. These can be used alone or in a combination of two or more.

For sodium chloride and calcium chloride, the salt concentration in the aqueous solution is about 0.1 to 4 w/v%, and preferably about 0.5 to 2 w/v%. For sodium gluconate and calcium gluconate, the salt concentration is about 1 to 34 w/v%, and preferably about 4.0 to 17 w/v%. For sodium lactate and calcium lactate, the salt concentration is about 0.1 to 10.0 w/v%, and preferably about 1.0 to 5.0 w/v%. For sodium malate and calcium malate, the salt concentration is about 0.1 to 15 w/v%, and preferably about 2.0 to 8.0 w/v%. For sodium citrate and calcium citrate, the salt concentration is about 0.1 to 25 w/v%, and preferably about 4.0 to 15.0 w/v%.

The concentration of sodium alginate in the aqueous solution is about 0.01 to 1.0 w/v%, and preferably about 0.1 to 0.6 w/v%. The concentration of gellan gum is about 0.01 to 1.0 w/v%, and preferably about 0.1 to 0.6 w/v%. The concentration of carrageenan is about 0.1 to 2.0 w/v%, and preferably about 0.5 to 1.5 w/v%.

The concentration of the polysaccharide aqueous solution used for the local injection solution is preferably within the above ranges since a sufficiently high mucosal elevation is obtained, the elevation is maintained for a sufficient period of time, and local injection is performed at an appropriate local injection pressure.

The injection volume of the polysaccharide aqueous solution into the submucosal layer is about 0.1 to 15 ml, and preferably about 1.0 to 5.0 ml.

The injection volume of the aqueous salt solution into the submucosal layer is about 0.1 to 10 ml, and preferably about 1.0 to 5.0 ml.

In the present invention, a new *ex vivo* model is created for evaluation of local injection solutions (*ex vivo* digestive tract sample for screening for local injection solutions for submucosal injection). In a conventional *ex vivo* model, evaluation was performed by fixing with multiple pins the circumference of a digestive tract sample, such as pig stomach section, on a stage, and measuring the height of the elevation obtained when a certain amount of a local injection solution was injected into the submucosal layer. Although this method appears to be a reasonable measurement, the height of the elevation varied greatly, even when the same local injection solution was injected. In contrast, the *ex vivo* model of the present invention shows small variations in data (small standard deviation), and enables detailed comparison between local injection solutions. The digestive tract sample may be a sample of the small intestine, large intestine, duodenum, stomach, or the like, preferably a stomach sample, and particularly preferably a pig stomach section. In the *ex vivo* digestive tract sample for screening for a local injection solution for submucosal injection of the present invention, a pig stomach section is placed on a stage and fixed with two or more fixing tools. The number of fixing tools is preferably 2 to 4, more preferably 2 to 3, and particularly preferably 2. The position of the fixing tools is not particularly limited, and they are preferably arranged in a symmetrical position. For example, when two fixing tools are used, the fixing tools are preferably arranged at opposing positions (on both sides) (Fig. 2). Examples of the fixing tool include a bulldog clip, a foldback clip, a chevron clip, a binder clip, and the like. As shown in Fig. 2, it is preferable that the ends of the digestive tract sample be completely sandwiched. In one embodiment of the present invention, a weight can be attached to one or more of the fixing tools to apply tension by hanging the weight outside the stage.

### Examples

The present invention is described in detail below with reference to Examples and Comparative Examples.

In the Examples, the following starting materials were used.
Sodium alginate:
   Sodium alginate, product name, produced by Nacalai Tesque, Inc.
Gellan gum:
   KELCOGEL (CG-LA), product name, produced by Sansho Co., Ltd.
Carrageenan:
   GENUVISCO (CF02), product name, produced by Sansho Co., Ltd.
Sodium hyaluronate:
   MucoUp, product name, produced by Seikagaku Corporation

### Production Example 1

The following polysaccharide aqueous solutions and aqueous salt solutions were prepared as local injection solutions.
- Polysaccharide aqueous solutions:
   Aqueous solutions of gellan gum (G-LA, 0.4%), hyaluronic acid (HA, 0.4%), sodium alginate (SA, 0.4%), or carrageenan (Agar, 1.0%) were prepared according to a conventional method.
- Aqueous salt solutions:
   Aqueous solutions of 0.9% NaCl or 2.0% CaCl₂ were prepared according to a conventional method.

These polysaccharide aqueous solutions and aqueous salt solutions were subjected to the following tests.

### Test Example 1

The local injection solutions obtained in Production Example 1 above were each injected into a newly developed *ex vivo* model for evaluation of local injection solutions using a pig stomach section shown in Fig. 2. The injection volumes were as follows:
- When the polysaccharide aqueous solution was used alone (SA, G-LA, Agar, or HA): 2.5 ml
- When the polysaccharide aqueous solution and the aqueous salt solution were used in combination (SA + Ca, G-LA + Ca, or Agar + Ca): 2.0 ml of the polysaccharide aqueous solution (SA 0.4%, G-LA 0.4%, or Agar 1.0%) + 0.5 ml of the aqueous salt solution
- Saline (NS): 2.5 ml

When the polysaccharide aqueous solution was combined with the aqueous salt solution, the polysaccharide aqueous solution was locally injected first, and then the aqueous salt solution was locally injected.

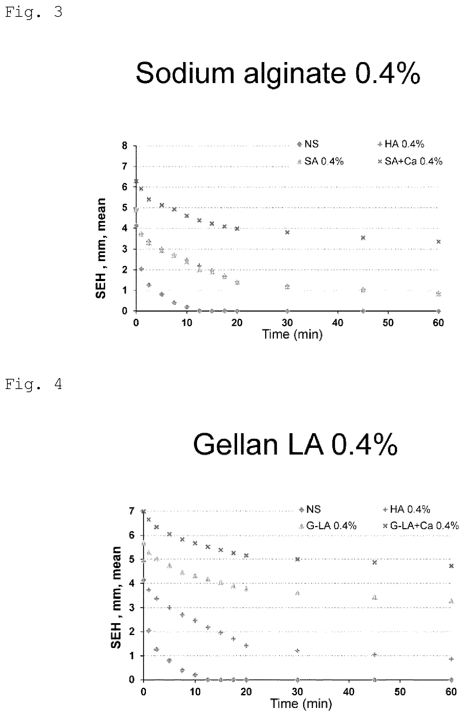

Fig. 3 is a graph showing the results of the measurement of the submucosal elevation height (SEH) when sodium alginate (SA, 0.4%) + calcium chloride (2.0%); sodium alginate (SA, 0.4%); hyaluronic acid (HA, 0.4%); and saline (NS) were each individually injected locally into the newly developed *ex vivo* model for evaluation of local injection solutions.

Fig. 4 is a graph showing the results of the measurement of the submucosal elevation height (SEH) when gellan gum (G-LA, 0.4%) + calcium chloride (2.0%); gellan gum (G-LA, 0.4%); hyaluronic acid (HA, 0.4%); and saline (NS) were each individually injected locally into the newly developed *ex vivo* model for evaluation of local injection solutions.

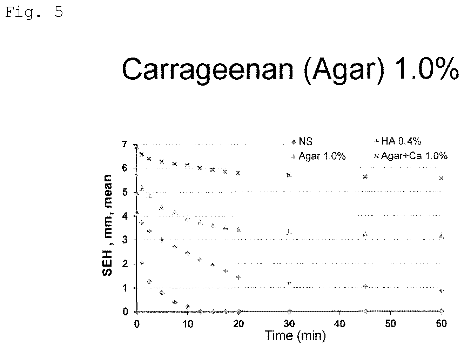

Fig. 5 is a graph showing the results of the measurement of the submucosal elevation height (SEH) when carrageenan (Agar, 1.0%) + calcium chloride (2.0%); carrageenan (Agar, 1.0%); hyaluronic acid (HA, 0.4%); and saline (NS) were each individually injected locally into the newly developed *ex vivo* model for evaluation of local injection solutions.

The results shown in Figs. 3 to 5 revealed that the two-component local injection solutions for submucosal injection of the present invention are excellent as a local injection solution for endoscopic treatment, since the use thereof enabled a high submucosal elevation height (SEH) to be maintained for a sufficient period of time.

## Claims

1. A two-component local injection solution for submucosal injection, comprising the following (i) and (ii):
(i) a polysaccharide aqueous solution containing at least one member selected from the group consisting of sodium alginate, carrageenan, and gellan gum; and
(ii) an aqueous salt solution containing at least one cation selected from the group consisting of sodium ions and calcium ions,
wherein when the polysaccharide is sodium alginate, the cation comprises calcium ions.

2. The two-component local injection solution for submucosal injection according to claim 1, wherein the polysaccharide concentration is 0.1 to 2% by mass.

3. The two-component local injection solution for submucosal injection according to claim 1 or 2, wherein the salt concentration is 0.1 to 4 w/v%.

4. The two-component local injection solution for submucosal injection according to any one of claims 1 to 3, wherein the polysaccharide is sodium alginate, and the aqueous salt solution contains calcium ions.

5. The two-component local injection solution for submucosal injection according to any one of claims 1 to 3, wherein the polysaccharide is carrageenan or gellan gum, and the aqueous salt solution contains sodium ions or calcium ions.

6. The two-component local injection solution for submucosal injection according to any one of claims 1 to 3 and 5, wherein the carrageenan comprises κ-carrageenan and/or ι-carrageenan.

7. The two-component local injection solution for submucosal injection according to any one of claims 1 to 6, which is for use in endoscopic mucosal resection (EMR) or endoscopic submucosal dissection (ESD).

8. Use of a polysaccharide aqueous solution containing at least one member selected from the group consisting of sodium alginate, carrageenan, and gellan gum, in the manufacture of a two-component local injection solution for submucosal injection.

9. Use of an aqueous salt solution containing at least one cation selected from the group consisting of sodium ions and calcium ions, in the manufacture of a two-component local injection solution for submucosal injection.
